# EUROPEAN PATENT SPECIFICATION

(11) **EP 1 474 973 B1**
(45) Date of publication and mention of the grant of the patent: **03.01.2007**
(21) Application number: 03705061.4
(22) Date of filing: 12.02.2003
(51) Int. Cl.: A01K 67/00, A61B 5/00, G01N 33/50, G01N 33/15, A01K 29/00, A61B 5/11

(54) **AUTOMATIC ANIMAL MOTION OBSERVATION METHOD AND APPARATUS, AND MOTION QUANTIZATION APPARATUS**
AUTOMATISCHES TIERBEWEGUNGSBEOBACHTUNGSVERFAHREN UND VORRICHTUNG DAFÜR SOWIE BEWEGUNGSQUANTISIERUNGSVORRICHTUNG
PROCEDE ET APPAREIL D'OBSERVATION AUTOMATIQUE DU MOUVEMENT D'UN ANIMAL, ET APPAREIL DE QUANTIFICATION DU MOUVEMENT

(30) Priority: 13.02.2002 JP 2002035266
(43) Date of publication of application: 10.11.2004
(73) Proprietor: Tokyo University of Agriculture and Technology Tlo Co., Ltd., Koganei-shi, Tokyo 184-0011 (JP)
(72) Inventor: MATSUDA, H. Agriculture Dpt., TOKYO UNIVERSITY OF, Fuchu-shi, Tokyo 183-8509 (JP); FURUMOTO, Akira c/o DIGIMO CORPORATION, Osaka-shi, Osaka 540-0019 (JP); MARUI, Tomohiro Engineering Dpt., TOKYO UNIVERSITY, Koganei-shi, Tokyo 184-8588 (JP)
(74) Representative: Schäfer, Matthias W.
(86) International application number: PCT/JP2003/001413
(87) International publication number: WO 2003/067973

(56) References cited:
- JP-A- 11 235 328
- JP-A- 61 083 964
- JP-A- 2000 055 906
- US-A- 4 917 117
- US-A- 5 608 209
- US-A- 5 826 578

## Description

### Technical Field

The present invention is to provide technology for quantifying specific movements/motions of organisms in relation to automation of observation of pharmaceutical tests by using experimental organisms; and more specifically, for quantifying the movements/motions of even nocturnal experimental animals.

### Background Art

Screening tests for minutely observing movements and behaviors of many model animals as experimental objects are conducted in order to increase statistical reliability of data in pharmaceutical tests using experimental animals, especially pathological model animals. In these screening tests, it is required to have clear indicators (objectivity) and ability to make quantitative decisions about whether medicaments, whose clinical effects are to be tested, are effective or not in the indicators. This is why quantification of specific movements/motions of organisms is indispensable. These screening tests should be preferably as simple and inexpensive as possible.

There are problems about methods of the screening tests for identifying the movements/motions of the animals through observations by human naked eyes, or for counting the numbers of times of certain motions of the animals through human observations. In other words, discrimination of the movements of the animals such as scratching, sneezing and sniffling is a long-time demanding labor requiring a lot of experiences, and a problem of objectivity and labor cost arises; therefore, automation of such observation is desired. However, all conventional methods and pieces of apparatus for the automation have their disadvantages. The conventional technology for automating observation of the movements of the animals is detailed, and the respective problems of such technology are explained hereinafter.

Japanese Patent Laid-open No. Hei 10-019658 publication, "Device for Observing and Analyzing Behaviors of Animals", discloses a device equipped a vibration sensor on a bottom of an animal cage for automatically recording kinds of movements of animals through detected vibrations. A whole picture can be obtained from the vibrations according to this publication, however, the kinds of movements/motions are hardly distinguished. Technology using a light sensor instead of the vibration sensor is explained hereinafter.

Disclosed are "Method for Measuring Quantity of Movements of Experimental Animals and Apparatus for the Same" (Toyo Sangyo Co., Ltd.) in Japanese Patent Laid-open No. Hei 07-184513 publication, "Process and Apparatus for the Recording and/or Evaluation of the Movement Behavior of Experimental Animals" (Ciba-Geigy Corporation) in Japanese Patent Laid-open No. Sho 63-074436 publication, both methods for measuring quantity of movements of experimental animals without touching them by the light sensor of a light projecting/receiving/shielding type, and "Method for Measuring Freezing Reactions and Device for the Same, and Method for Evaluating Influence of Chemical Substances Affected to Learning or Memory" (The Institute of Environmental Toxicology) of Japanese Patent No. 2969108, the method for objectively measuring freezing reactions of animals by using a passive far infrared sensor in which wavelengths of the light sensor are set to far infrared regions so that animal temperature information can be also incorporated. "Apparatus of Compulsory Swimming Test for Experimental Animals" (Muromachi Kikai Co., Ltd.) described in Japanese Patent Laid-open No. Hei 10-197345 publication, the apparatus for detecting change of temperature distribution of the experimental animals and the like in a test tank by similarly using an infrared sensor, and for objectively evaluating effects of antidepressants by determining energy of the animals according to the change, or the like is also listed.

However, when adopting such technology, fine distinctions of the kinds of movements/motions are hardly distinguished because little information is obtained from the light sensor as mentioned above. Furthermore, a problem that key movements/motions of the animals may fail to be detected depending on ability of the light sensor and installation conditions thereof arises.

In order to incorporate information to draw even the fine distinctions of the kinds of the movements, there is a method for video-recording the experimental animals and for analyzing dynamic images by adopting various kinds of image analysis algorithms. For example, "Method for Automating Observation of Behaviors of Experimental Animals" (Chuo Electronics Co., Ltd., et. al.) described in Japanese Patent Laid-open No. Hei 11-296651 publication discloses a method for extracting image parameters by carrying out image processing constituted by a moving target indication, a binarization method, an ellipse approximation, and a centroid extraction, and for discriminating behaviors of animals such as locomotion, stretching, rearing, immobility, sniffing, and grooming.

However, this method of the image analysis handles an excessive amount of information of moving images, which requires a lot of time for processing information. Therefore, less amount of information processing than that of the aforementioned method and more information than insufficient information in the method such as the methods of the vibration sensor of the floor on the animal cage and the light sensor of the light projecting/receiving/shielding type, which means only necessary and sufficient movements/motions information for quantifying, should be incorporated and processed.

For example, information incorporation should be limited to only parts required for quantifying the movements/motions such as muscles, joints, and the like so that the moving image of only the parts is incorporated by a video or the like. However, conventionally, there was no appropriate method.

Meanwhile, there is a method for attaching plural position markers such as luminescent materials, magnetic substances, or the like on organisms (human bodies), and for digitalizing movements by them, which is generally known as motion capture technology. The motion capture technology is utilized in various science fields such as biomechanics, rehabilitation therapy, sport science, industrial use of virtual reality, and the like.

In the motion capture technology, the plural position markers such as the luminescent materials, the magnetic substances, or the like are attached on an actor (a movements/motions performer) such as the human bodies, the organisms, or the like, and a dynamic image is video-recorded. Then, the recorded movements are replayed and analyzed according to the data. The position markers include magnetic markers, optical markers, those of electromyogram methods, and the like. As one of the optical markers, color markers for discriminating markers by colors are disclosed in "Motion Analyzer" (Anima Co., Ltd.) described in Japanese Patent Laid-open No. Hei 04-93704 publication, and "Apparatus for Analyzing Behavior of Parts of Subjects" (Anima Co., Ltd.) described in Japanese Patent Laid-open No. Hei 06-50983 publication, and the like.

The motion capture technology aims at replay and analysis of three-dimensional movements/motions. Information processing of it requires a lot of time because complicated information processing is carried out by incorporating a lot of data from many position markers. Therefore, this technology is not applicable to automating observation of the experimental animals. Furthermore, attachment of many of the position markers such as the luminescent materials, the magnetic substances, or the like to animals brings a problem of injury to the animals when attaching them, and causes stress to the animals after the attachment, therefore the attachment is inappropriate. Unnecessary stress to the animals should never exist for observation of especially an atopy pruritus behavior or the like. Moreover, markers generally known are useless because they are easily detached due to pruritus behaviors or the like of the animals.

On the other hand, nocturnal animals such as mice, cats, bats, and the like are used for various kinds of animal experiments and animal studies in some cases. There is a condition that such animals must be experimented under night vision environments because they do not behave naturally in the light. Generally, a night vision camera (an infrared camera) is used for observing behaviors of the nocturnal animals as disclosed in "Detector for Passing of Wild Animal" (Sanyo Sokki Co., Ltd.) described in Japanese Patent Laid-open No. Hei. 11-306313 publication. However, only moving images are obtained from the night vision camera, and a lot of time is required for analyzing movements/motions like the above-mentioned methods. Therefore, this is not applicable to automating observation.

According to the present invention, fluorescence by ultraviolet light is used in order to solve the above-mentioned problems of the experiments under the aforementioned night vision environments. The ultraviolet light is also called a black light. A fluorescent material emits light with a visible wavelength by receiving invisible light in an ultraviolet wavelength region, which is a principle of a fluorescent light. Approximately 340 nm of the wavelength region is used for an ultraviolet ray which generates fluorescence. Any fluorescer which generates the fluorescence by irradiating with the ultraviolet ray is acceptable. For example, the fluorescer among commercially available rhodamine, auramine, and the like is selected considering color tones of emitted light, toxicity, and the like, and depending on purposes.

Techniques in which the fluorescence is applied to the organisms include "Apparatus for Automatically Determining Freshness of Hen Eggs" (Kyowa Machinery Co., Ltd.) described in Japanese Patent Laid-open No. Hei 11-230960 publication, the apparatus for determining freshness of hen eggs by irradiating the hen eggs with the ultraviolet ray and by carrying out image processing of fluorescence emitted from the hen eggs, and "Method for Treating Water by Biological Activated Carbon and System for the Same" (Hitachi Ltd.) described in Japanese Patent Laid-open No. Hei 08-252587 publication, the method for irradiating treated concentrate water in a biological activated carbon tub with the ultraviolet light, for carrying out image processing of the fluorescence the organisms emit, and for determining performance deterioration of the biological activated carbon, or the like.

Furthermore, "Method for Detecting Tracking Abnormality" (NKK Corporation) described in Japanese Patent Laid-open No. Hei 07-149420 publication, the method for utilizing fluorescence as a tracking material to be processed, and "Method for Searching Animal Path" (Ikari Shodoku Co., Ltd.) described in Japanese Patent Laid-open No. Hei. 10-111364 publication, the method for feeding fluorescent materials to the animals and for tracking excrement of the animals by irradiating the excrement with the ultraviolet ray so as to emit fluorescence as a utilization for tracking the animals, and the like are laid open.

An object of the present invention is, in various kinds of experiments using organisms, to provide a method and an apparatus for carrying out processing of only necessary and sufficient information which needs quantification of movements/motions, and for obtaining quantitative experimental results within a practical time of information processing, and also a method for collecting data of movements/motions with no injury or stress to organisms; and more specifically, a method and an apparatus for collecting data of nocturnal animals such as mice, cats, bats, and the like in a dark environment where they behave naturally.

### Disclosure of the Invention

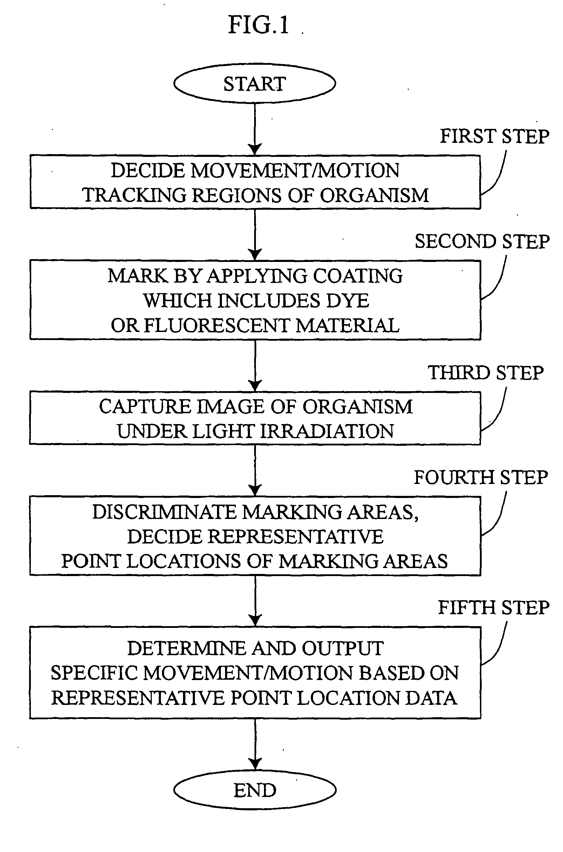

This invention is a method for quantifying specific movements/motions of organisms by applying coating which includes a dye or a fluorescent material to plural movement/motion tracking regions on surfaces of the organisms decided based on the movements/motions . FIG. 1 shows a flow chart of work operation of the method according to this invention. This invention comprises a first step for deciding affected parts of the organisms and portions of the organisms with which the organisms act on the affected parts as movement/motion tracking regions.

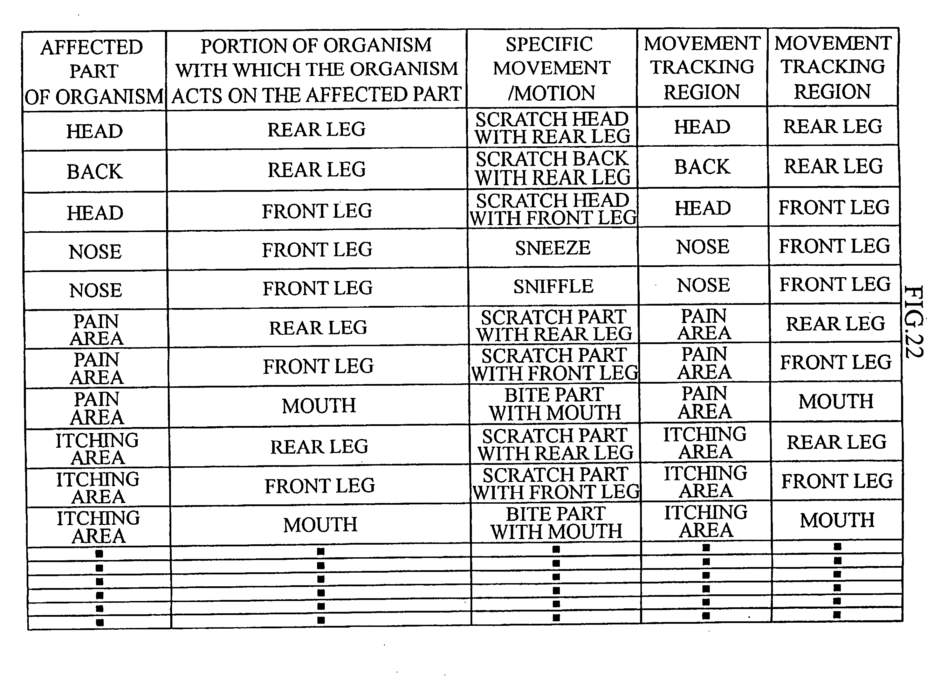

FIG.22 shows a chart explaining a relationship between the affected parts of the organisms, the portions with which the organisms act on the affected parts, the specific movements/motions, and the movement/motion tracking regions. For example, if the affected part is a head and the specific movement/motion is scratching behavior on the head with a rear leg, the head and the rear leg are the movement/motion tracking regions. Other examples shown in FIG. 22 may be referred accordingly in the course of a following explanation.

After the first step for deciding the aforementioned movement/motion tracking regions, a second step for marking the movement/motion tracking regions by applying coating which includes a dye or a fluorescent material, a third step for capturing an image of the organisms under light irradiation, a fourth step for discriminating marking areas in a captured image and for deciding representative point locations of the marking areas, and a fifth step for determining the specific movements/motions based on plural representative point location data and for outputting the number of times of the specific movements/motions are carried out. Observation is automated by quantifying the specific movements/motions of the organisms through these steps.

Organism surfaces mean surfaces of the organisms literally. These surfaces include skin tissues, cell tissues, and may be scale tissues for fish and reptiles. Hair and fur may be included because the hair is a transformed object of skin. Portions differentiated into sense organs or keratinized surfaces may also be included. Therefore, the movement/motion tracking regions of the organism surfaces may be a collection of the skin, the hair, or the fur, or may be the scale tissues, or the portions differentiated into sense organs. The respective steps in pharmaceutical tests using the animals are explained hereinafter.

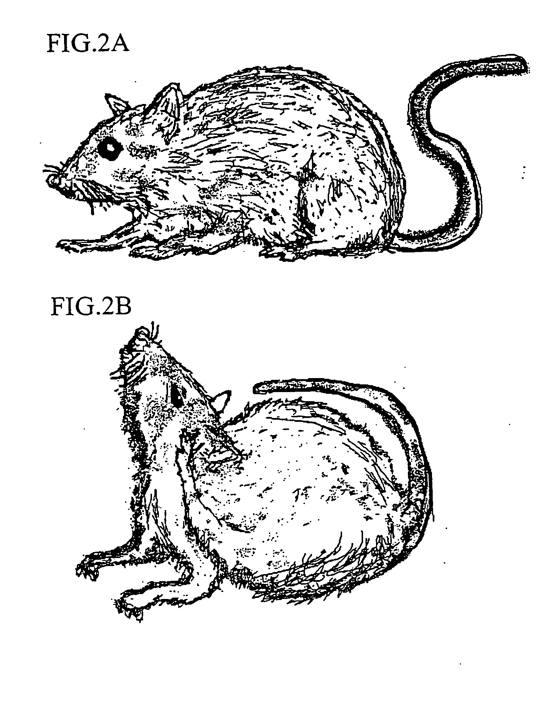

For example, NC/Nga mice are known as model animals which naturally develop atopic dermatitis. FIGS. 2A and 2B show the mice which develop this disease. A pharmaceutical test is conducted by giving a new medicine of atopic dermatitis to one of the mice which develop the disease and a placebo to the other and by observing their pruritic behaviors. Behaviors to be observed at this time are the number of scratch behaviors on their heads with their rear legs as shown in FIG. 2B. Conventionally, the number of times was counted by judging whether the behaviors occurred or not through observations by naked eyes. Furthermore, motion capture technology has not been applied to such a method of counting the number of times.

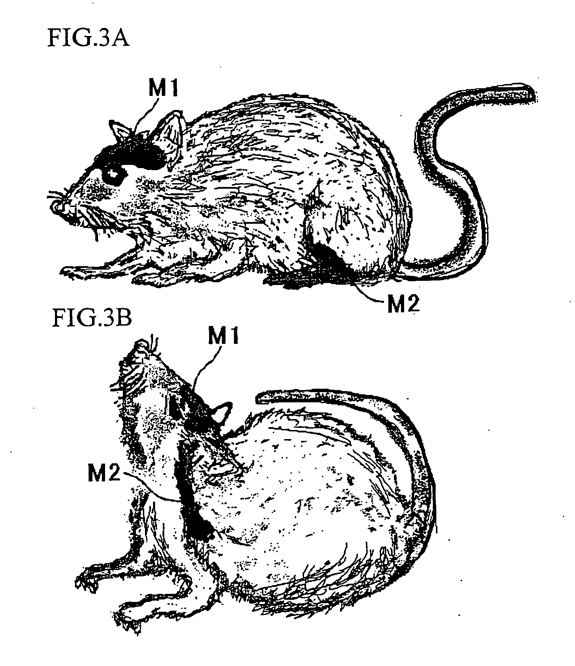

In this example, the affected part of atopic dermatitis is the head, and the specific movements/motions occur by scratching the head as the affected part with their rear leg as the portion of the organism. One of movement/motion tracking regions is considered to be, for example, a top of the head, and the other, for example, a top of the rear leg. Whether the scratching behaviors occur or not is determined by approach of these tracking regions. As shown in FIGS. 3A and 3B, red coating is applied to portions M1 which cover the heads infinitely, and green coating to portions M2 which cover the tops of the rear legs infinitely (the second step).

Coating should be applied to the mice after giving them anesthesia so that they are not injured or do not suffer stress. Coating materials containing natural dye and the like as main ingredients should be used considering safety to the mice. Application of "sticky" ingredients to front legs or the rear legs will cause stress to the animals as their general habit; therefore, quick-dry coating materials should be applied to the rear legs considering such habit.

Experiments are conducted by observing the scratching behaviors such as scratching the head with the rear leg on model animals which develop not only atopic dermatitis but also general dermatological diseases. Scratching behaviors are not limited to scratching the head with the rear leg. Some animals scratch themselves with the front legs. The affected parts are often backs. Therefore, the movement/motion tracking regions should be divided into the heads, the backs, the front legs, and the rear legs according to the affected parts and behaviors of the animals (see FIG. 22).

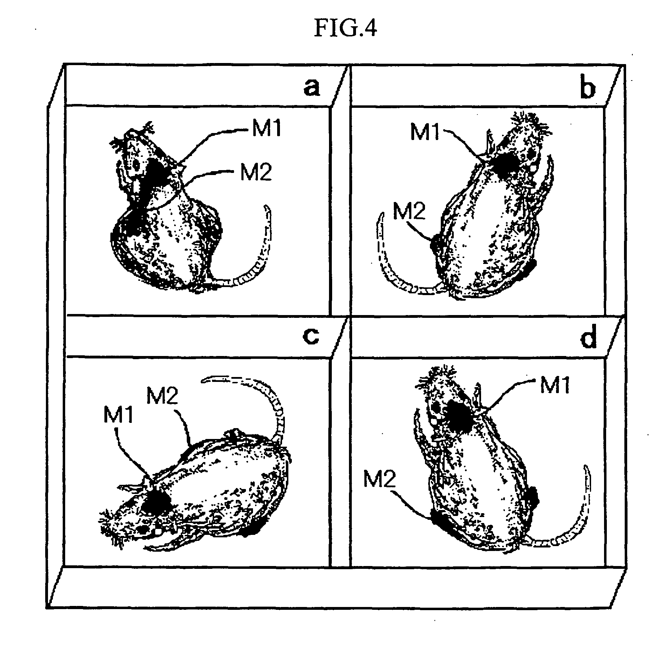

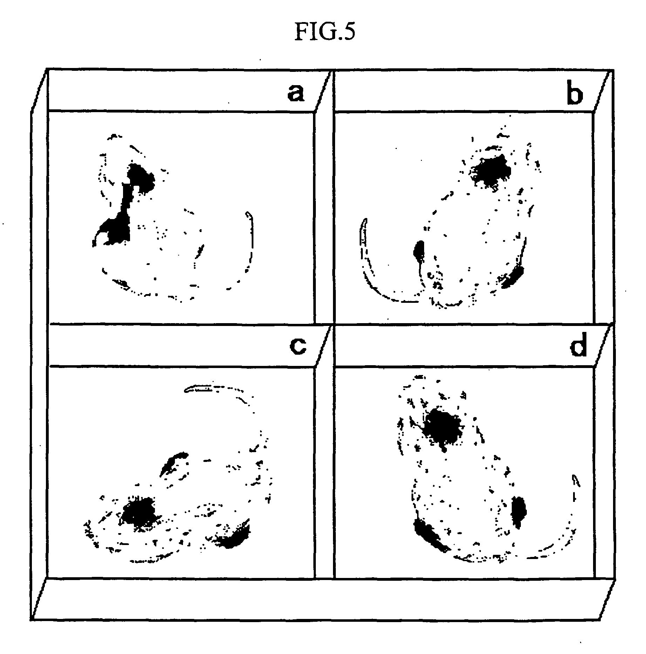

Four mice to which dye coating was thus applied are moved to individual cages, and recorded by a digital video camera under visual light (the third step). A captured image of them is schematically shown in FIG. 4. Fluorescent coating is applied on the portions M1 and the portions M2 of the respective four mice, and they are moved to cages in a dark room. They are recorded using a digital video by irradiating the cages with the ultraviolet ray of a black light so that the fluorescent coating emits visual light. A captured image of them is schematically shown in FIG. 5.

The images that the portions M1 and the portions M2 can be visually discriminated well by a commercially available digital video camera were obtained in both cases of the former dye coating and the latter fluorescent coating. Especially in the case of the latter fluorescent coating, the portions M1 and the portions M2 are easily discriminated due to clear contrast. Since the mice are nocturnal animals, they behave actively and naturally in the dark room under observation, which is desirable for the pharmaceutical tests. This means that the third step should be preferably conducted in the dark room and the like under irradiation with ultraviolet light by using the fluorescent coating as coating if study animals are nocturnal.

Image data of a digital camera includes color RGB (red, green, blue) data of pixels in each image frame, and is available with position coordinate data of the pixels. Similarly, luminance (optical power) data of the pixels in each of the image frames is also included and is also available with the position coordinate data of the pixels.

It is easy to discriminate and extract coordinates of the portions M1 and the portions M2 in the image by the color or luminance data. For example, red coating is applied on the portions M1, so that the marking areas M1 can be extracted by extracting portions where R (red) data values are large. This is a sub-step for discriminating the marking areas in the image by applying coating in the fourth step.

The plural portions M1 and the plural portions M2 are mingled in the images shown in FIGS. 4 and 5; however, locations of the individual cages (a), (b), (c), and (d) in the drawings are fixed. Therefore, the portions M1 and the portions M2 of the respective cages can be discriminated if the areas
where the R (red) data values are large are considered as the portions M1 and areas where G (green) data values are large as the portions M2, with the conditions that they are positioned within the respective cages. It is acceptable to apply different colors such as red, blue, green, yellow, and the like to the respective mice (a), (b), (c), and (d) for discrimination, and it is possible to apply different colors to the respective marking areas for distinction. Even if three or more of the marking areas are on one organism, they can be easily discriminated by thus changing colors. Also in a case of irradiating with the ultraviolet ray such as the black light so as to emit fluorescent light, discrimination by color is possible like the aforementioned method because visually recognizable luminescent colors change by changing fluorescent materials to apply.

Since the luminance (the optical power) data of the pixels is also included in each image frame, it is acceptable to discriminate by only the difference between the luminance data of the pixels, or also acceptable to discriminate by combining the color data with the luminance data. The data obtained by capturing the image is changed according to experimental conditions such as size and locations of lights, and the like. Discrimination is possible if the luminance and color data of the marking areas as targets obtained by the experiments are measured by pre-experiments. Needless to say that the coating or the like does not have to be necessarily applied to the mice if the experimental conditions are good, where characteristics of the marking areas on the image are noticeable and discrimination of the marking areas is possible by only image processing. For example, coating is not required if the characteristics of eyes, the front legs, or the rear legs on the image are extracted by only image processing.

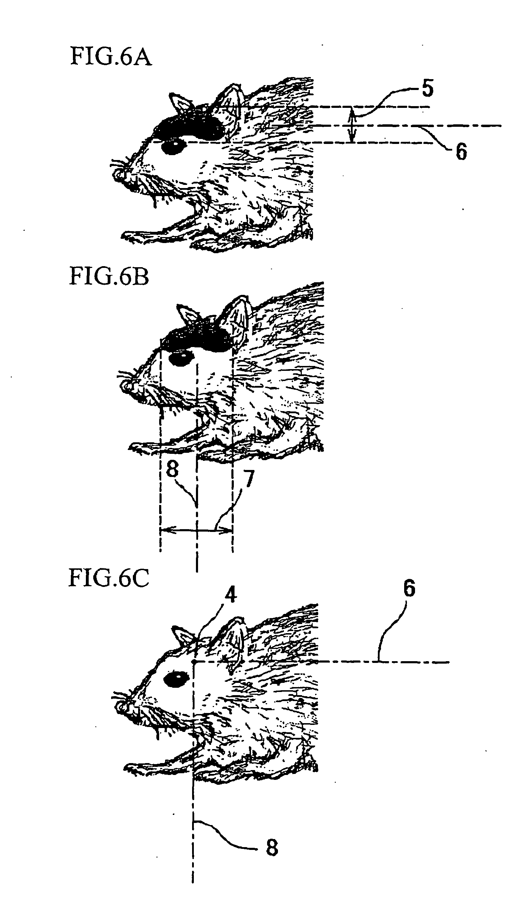

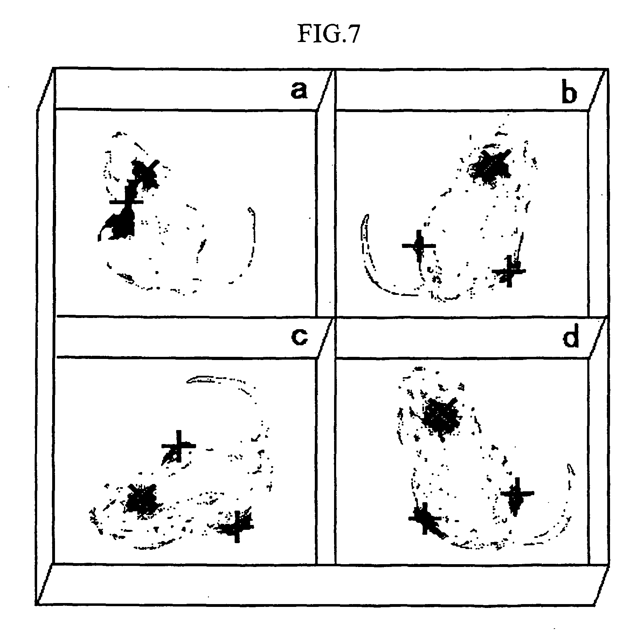

Next, a sub-step for deciding representative point locations of the marking areas in the fourth step is explained hereinafter. FIGS. 6A to 6C show views of examples in which a point is decided from the images of the marked mice. Among all pixel coordinates on the images of the corresponding portions M1 and portions M2 specified by the color or luminance data, a maximum coordinate and a minimum coordinate thereof are extracted, and central points of them are considered to be the representative points. This means that a midpoint 6 of a vertical coordinate is calculated from a width 5 (the maximum coordinate and the minimum coordinate) of the vertical coordinate as shown in FIG. 6A, and a midpoint 8 of a horizontal coordinate from a width 7 (the maximum coordinate and the minimum coordinate) of the horizontal coordinate as shown in FIG. 6B, so that a representative point 4 can be determined. The image shown in FIG. 5, on which the representative points "x" and "+" are added, is shown in FIG. 7 as a reference.

With regards to image processing technology, many methods for carrying out the two sub-steps of the aforementioned fourth step, that is, the sub-step for discriminating the marking areas and the sub-step for deciding the representative point locations are generally known, and any of them can be employed. Specifically, the image processing methods such as a moving target indication, a binarization method, an ellipse approximation, a centroid extraction, and the like may be employed as necessary.

Next, the fifth step for determining the specific movements/motions based on the decided plural representative point location data and for outputting them is explained hereinafter. It is easy to calculate the distances between the representative points "x" and "+" shown in FIG. 7 because the coordinate data is already known. Flat distances in flat images are calculated here. If necessary, distances which are closer to absolute distances can be obtained by calculating data of three-dimensional representative points with plural cameras with the use of the motion capture technology publicly known. However, the flat distances have no practical problems.

In case that marking areas are sometimes out of sight of the images, since the animals frequently move around, processing can be carried out in a time on the images except when the marking areas are out of sight. That is, the number of the marking areas is already known; therefore, if the number of the representative points is below the number of the marking areas, the captured image data may be excluded, or the like. For example, if both of the images of the mouse to which a new medicine was administered and the mouse to which a placebo was administered are excluded at the same time on the image, and processing is carried out only when all of the marking areas of both mice are in sight, most of the time on the images can be in use and have no practical problems.

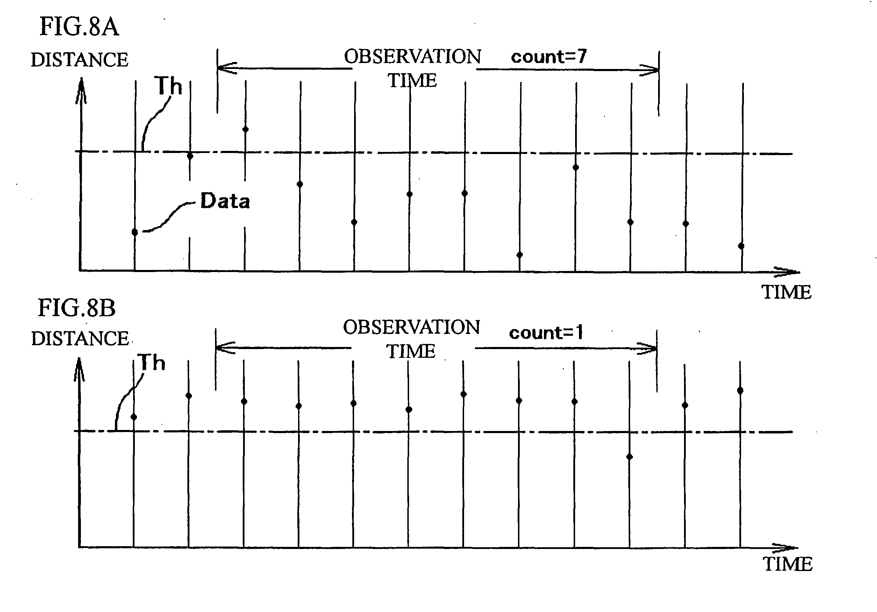

The distances between the representative points are changed according to the behaviors of the animals. In FIGS. 8A and 8B, data (Data) of the distance between the representative points are plotted considering a horizontal axis as a time axis. Appearance of the scratching behaviors is recognized when the portion M1 which infinitely covers the head and the portion M2 which infinitely covers the rear leg are approached. Accordingly, the appearance of the scratching behaviors can be recognized if the distances between the representative points of them are quite small values. Therefore, condition distances for determining the movements should be decided in advance and determination of the appearance of the movements can be made if the distances are below the condition distances. A value Th shown in FIGS. 8A and 8B is a level representing distance condition data decided in advance. The value Th is considered to be a threshold value for determining whether the movements occur or not.

The condition distance Th is not absolutely decided. However, for example, the experiments are evaluated by the difference between the movements of the mouse to which the new medicine was administered and the mouse to which the placebo was administered; therefore, relative evaluation of new medicines can be performed if the respective motion determining condition distances Th are the same. Therefore, the condition distance Th can be accordingly set up for the experiments according to individual differences of the mice, conditions of the marked coating, video-recording conditions, or the like. Furthermore, they can also be determined by combining observation by naked eyes or the video image of the movements of the mice with the calculated distance data at the beginning of the experiments. Slow-motion replay or the like of the video can be utilized at this time.

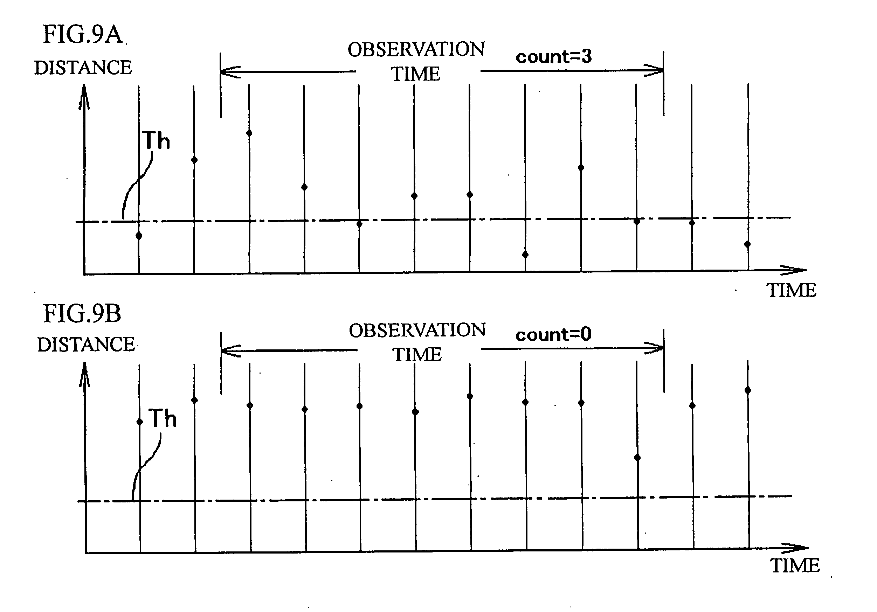

In the data graph of the mouse to which the placebo was administered as shown in FIG. 8A, during the observation time shown in the drawing, the values of the distance data (Data) went below the condition distance Th seven times, so seven movements were counted (count = 7). One movement was counted in the case of the mouse to which the new medicine was administered as shown in FIG. 8B (count = 1). The new medicine can be evaluated by comparison between these counts. FIGS. 9A and 9B show examples of cases when the values of the condition distances Th are lower than those in FIGS 8A and 8B. FIG. 9A shows three counts (count = 3), and FIG. 9B zero count (count = 0).

The observation time can be commonly set up with no restraint between the mouse to which the new medicine was administered and the mouse to which the placebo was administered. Time intervals of data collection can also be commonly set up with no restraint between the mouse to which the new medicine was administered and the mouse to which the placebo was administered. For example, graphs in which the time intervals of data collection are one third of that in the cases shown in FIGS. 8A, 8B, 9A, and 9B are respectively shown in FIGS. 10A, 10B, 11A, and 11B. An explanation of them is omitted.

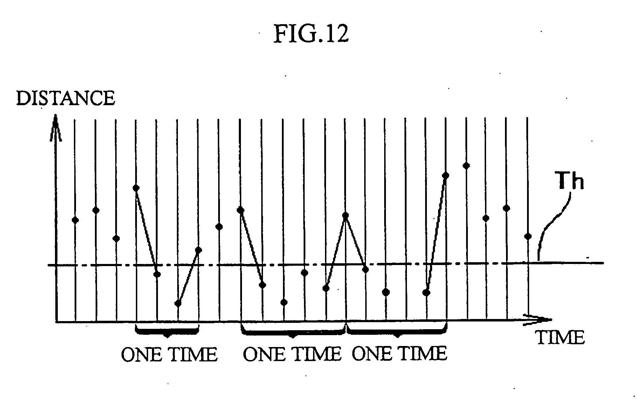

The decision of the movements is not limited to the aforementioned method for determining the appearance of the movements if the values of the distance data (Data) are the Th values or below, and for counting the values. For example, one count may be considered to occur if the value of the distance data (Data) are larger than the Th value and then go under the Th value. Furthermore, as shown in FIG. 12, one count may be considered to occur if the distance data (Data) value reciprocates by going under the Th value from a value larger than the Th and then coming back to the value larger than the Th.

Conventionally, the method for directly determining the movements/motions from information obtained as the moving images as described in the aforementioned Japanese Patent Laid-open No. Hei 11-296651 was proposed. In contrast, according to this invention, an amount of image processing is limited to only the marking areas which are a part of the moving images; furthermore, the movements are determined by only the aforementioned simple calculation of the distances. Therefore, processing time is dramatically shortened.

As described above, the method for automating observation relating to this invention was explained with reference to the model mice which naturally develop dermatitis. This is the example of the model animals which naturally develop the disease. In this case, the model animals naturally start scratching behaviors. In contrast, some behaviors may be induced by applying or administering irritants to the experimental animals to stimulate senses which make the specific movements/motions. That is, for example, by applying the irritants to the surfaces of the organisms or by administering the irritants to the organisms right before or right after the second step (coating application), the senses which make the specific movements/motions are stimulated, so that the behaviors are induced.

Pseudo-illness and tentatively affected parts are caused by the aforementioned application or administration to even sound organisms. The specific movements/motions are induced to the affected parts; therefore, it is easy to determine the affected parts, and portions where the organisms act on the affected parts as the movement/motion tracking regions.

For example, in the case of the pharmaceutical tests of the dermatological illness, irritants for itching senses such as histamine, serotonin, and the like are applied to the surfaces of the experimental animals or administered so as to make scratching behaviors to the affected parts, considering itching areas as the affected parts. On the other hand, in the case of the pharmaceutical tests of otolaryngologic illness or ophthalmic illness such as pollinosis, the irritants for the itching senses such as the histamine, the serotonin, and the like are applied to the noses of the experimental animals or administered so as to cause sneeze or sniffle behaviors, considering the noses as the affected parts.

Furthermore, animals have habit to bite pain areas or to scratch them with their front or rear legs. Therefore, in the case of pharmaceutical tests of new sedatives, irritants for aching senses such as formaldehyde, capsaicin, and the like are applied or administered to the experimental animals so as to induce bites of pain areas and scratches of them with their front or rear legs, considering the applied areas or specific organs and tissues influenced by the administrated irritants as the affected parts.

Here, in most of the cases of application of the irritants, the applied areas are considered to be the affected parts; therefore, the applied areas can be considered as the motion tracking regions. For example, in the pharmaceutical tests of the dermatological illness, one of the movement/motion tracking regions may be the back, and the other thereof the front leg or the rear leg by applying the histamine to the back.

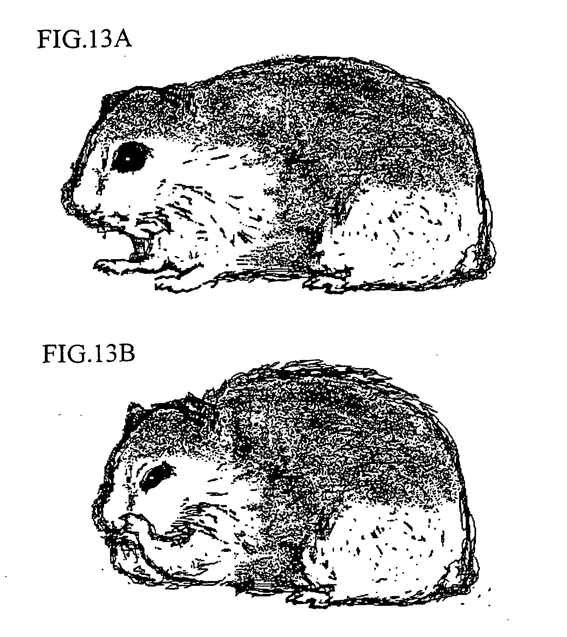

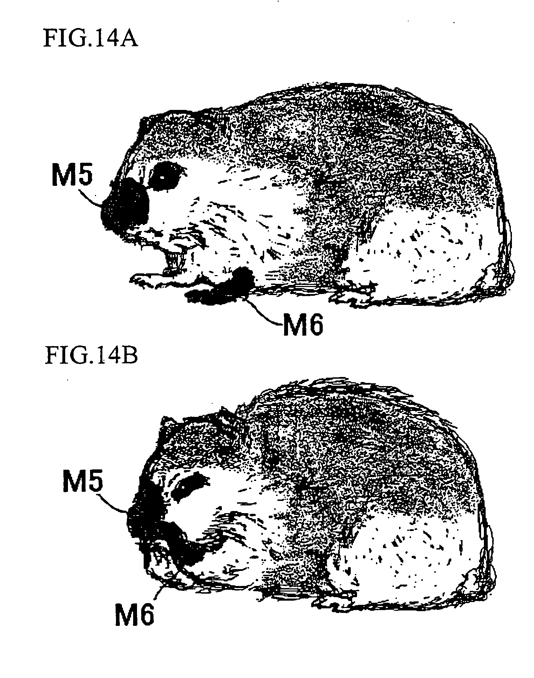

In the case of the pharmaceutical tests of otolaryngologic illness such as pollinosis, for example, the irritant is applied to the nose of a guinea pig shown in FIG. 13A so as to induce sneeze or sniffle behaviors shown in FIG. 13B. In this case, as shown in FIGS. 14A and 14B, the nose M5 and the front leg M6 to which the irritants were applied may be considered as the movement/motion tracking regions.

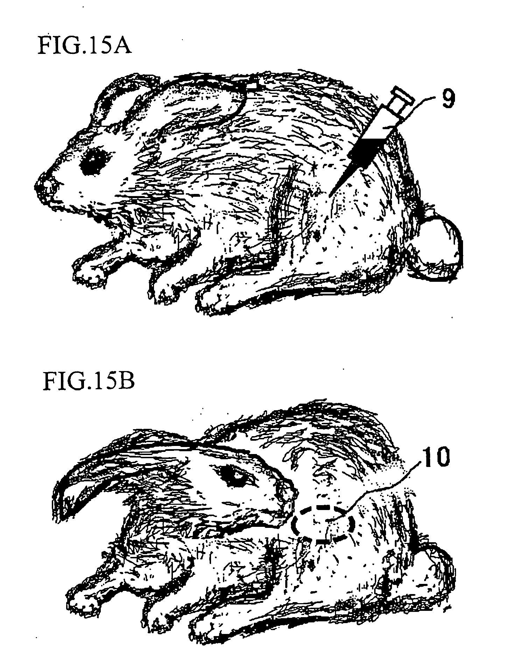

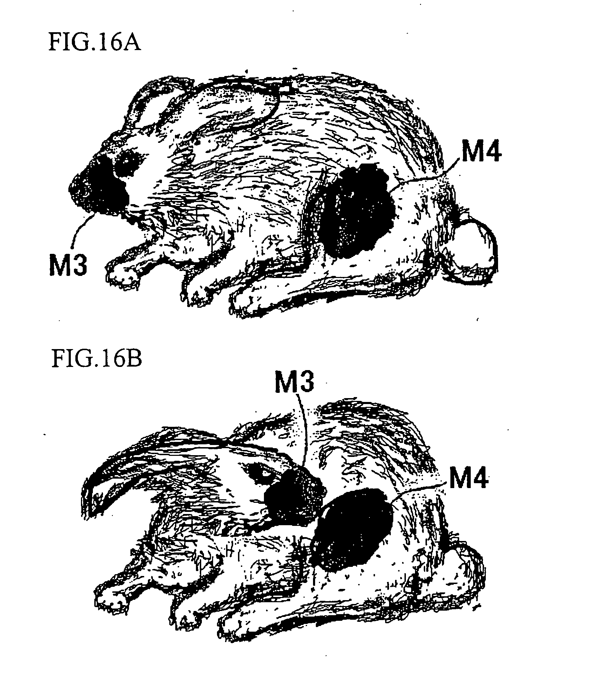

Here, administration of the irritants may be any of oral administration, administration by inhalation, and administration by injection. For example, in the case of the pharmaceutical tests of the new sedative, the irritant for aching is administered into one of the rear legs of an experimental rabbit by an injection syringe 9 as shown in FIG. 15A. After this, a biting behavior of an injection site 10 is induced as shown in FIG. 15B because the injection site under fur becomes the affected parts and irritated. Therefore, as shown in FIGS. 16A and 16B, coating may be applied to portions M3 which infinitely cover mouths and portions M4 which infinitely cover the injection sites as the aching parts.

The irritating areas by the administration of the irritants are not always limited to the administrated areas. For example, a substance for inducing headaches such as the formaldehyde or the like is administrated by inhalation so as to trigger headache irritation and induce scratch behaviors of the heads in some cases. In these cases, the affected parts are organism organs irritated by the administered irritants, that is, the heads (brains), and the movement/motion tracking regions are also the heads. Similarly, a substance for irritating mucous tissues such as the capsaicin and the like is orally administered so as to irritate pharyngeal mucosa and induce scratch behaviors of throats with the rear legs in some cases. In these cases, the affected parts are pharynxes and the movement/motion tracking regions can be also the throats which are the aching parts.

FIG. 22 shows the relationship described above between the areas of the affected parts of the organisms, the portions of the organisms with which the organisms act on the affected parts, the specific movements/motions, and the movement/motion tracking regions.

### Brief Description of Drawings

FIG. 1 is a flow chart of work operation of a method for automating observation of movements/motions of organisms relating to this invention;
FIGS. 2A and 2B are views explaining mice which are model animals of atopic dermatitis and a scratching behavior thereof;
FIGS. 3A and 3B are views of the mice whose heads and rear legs are marked;
FIG. 4 is a view showing an example of a captured image of the marked mice in respective four cages;
FIG. 5 is a view showing an example of a captured image of the fluor-marked mice under irradiation with an ultraviolet ray;
FIGS. 6A to 6C are views explaining a method for deciding a representative point from the images of the marked mice;
FIG. 7 is a view of an image in which the representative points are added to the image of FIG. 5;
FIGS. 8A and 8B are graphs of data of distances (intervals) between the representative points plotted on time axes (examples of high Th values);
FIGS. 9A and 9B are graphs of data of distances (intervals) between the representative points plotted on time axes (examples of low Th values);

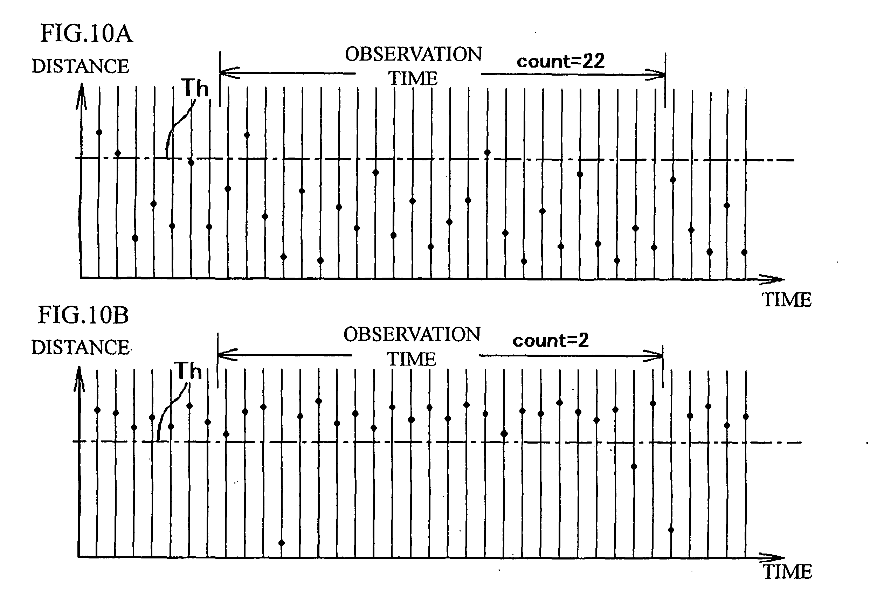

FIGS. 10A and 10B are data plot graphs in which time intervals of data collection are one third of that in the cases shown in FIGS. 8A and 8B;

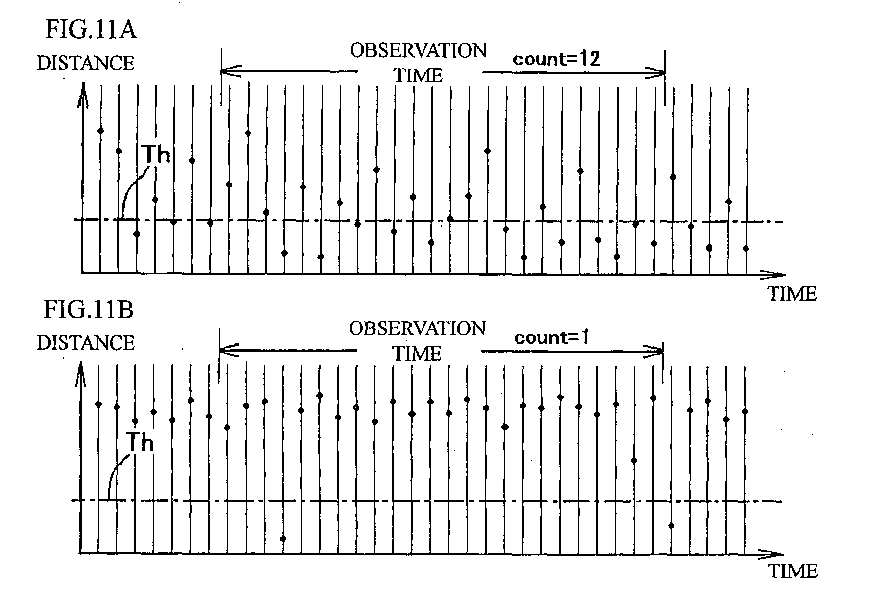

FIGS. 11A and 11B are data plot graphs in which time intervals of data collection are one third of that in the cases shown in FIGS. 9A and 9B;
FIG. 12 is a view explaining an example of a method for determining one time of the movements/motions;
FIGS. 13A and 13B are explanatory views of an experimental guinea pig for pharmaceutical tests of pollinosis, of a sneeze behavior and of a sniffle behavior;
FIGS. 14A and 14B are views of a guinea pig whose nose and front leg are marked by applying an irritant;
FIGS. 15A and 15B are views explaining experimental rabbits used for pharmaceutical tests of sedatives and a biting behavior of an aching part;
FIGS. 16A and 16B are views of a rabbit whose injection site of the irritant and mouth are marked;

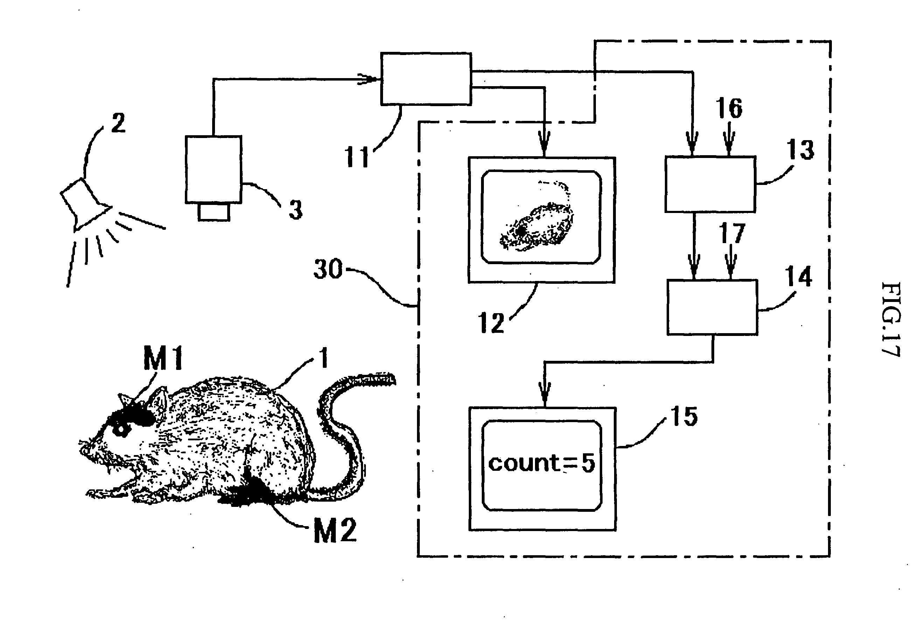

FIG. 17 is a structural view of an apparatus for automating observation of the movements/motions of organisms, and an apparatus for quantifying the specific movements/motions relating to an embodiment of this invention;

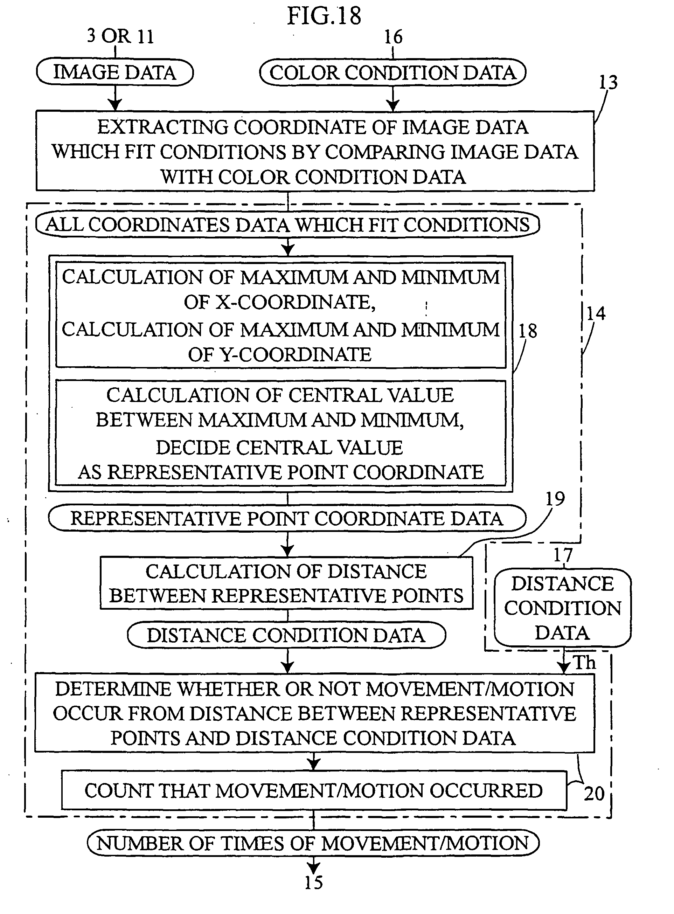

FIG. 18 is a view explaining a discriminating means 13 and a movement/motion counting means 14;

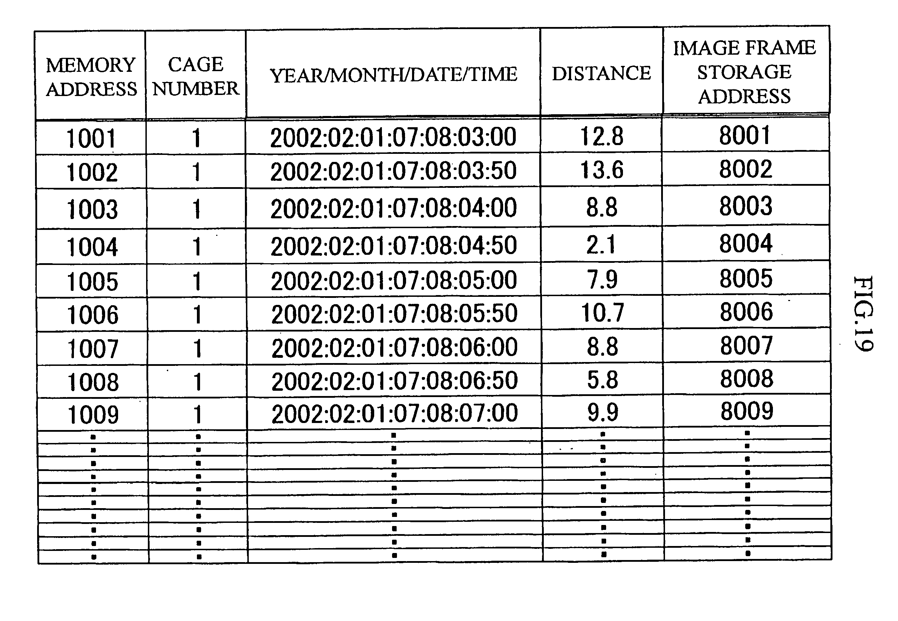

FIG. 19 is a chart showing a part of data of a cage number 1 (coordinate data and color data are stored in an image frame address);

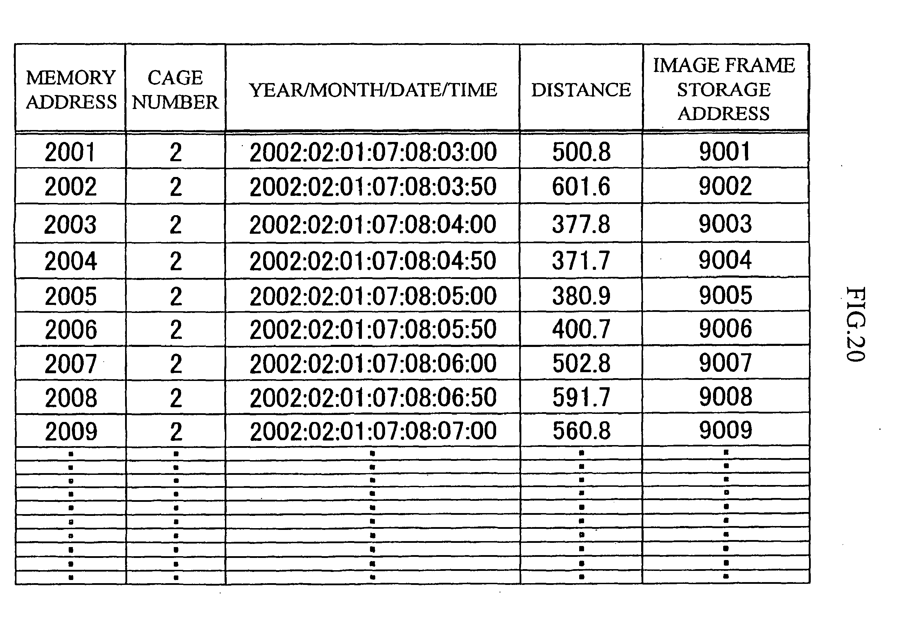

FIG. 20 is a chart showing a part of data of a cage number 2 (coordinate data and color data are stored in an image frame address);

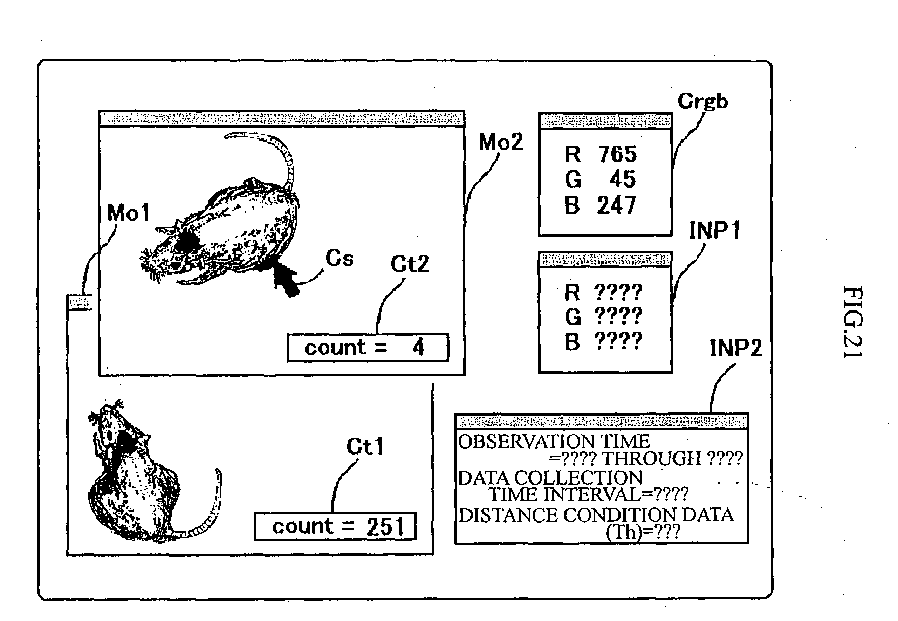

FIG. 21 is a view showing a display example of the apparatus (a personal computer) for quantifying the specific movements/motions relating to the embodiment of this invention; and
FIG. 22 is a chart explaining a relationship between affected parts of the organism, portions of the organisms with which the organisms act on the affected parts, specific movements/motions, and movement/motion tracking regions.

### Best Mode for Carrying out the Invention

An apparatus for automating observation by quantifying specific movements/motions of organisms relating to an embodiment of this invention is explained in FIGS. 17 and 18. This apparatus is the apparatus for quantifying the specific movements/motions of organisms by applying coating which includes a dye or a fluorescent material to plural movement/motion tracking regions on surfaces of the organisms decided based on the movements/motions. It is a prerequisite to have decided the movement/motion tracking regions as shown in FIG. 22, for examples.

This apparatus is characterized in that it includes an irradiating means 2 for irradiating, with light, an organism 1 to which coating including a dye or a fluorescent material is applied, as M1 and M2 in FIG. 17, on both an affected part of the organism and a portion of the organism with which the animal acts on the affected part decided as movement/motion tracking regions, a capturing means 3 for capturing an image of the organism under the irradiating means, a discriminating means 13 for discriminating coating-applied areas from captured image data and discriminating condition data (an external setting input 16 in FIG. 17), and a movement/motion counting means 14 for determining specific movements/motions from both coordinate data of the discriminated coating-applied area and motion determining condition data (an external setting input 17 in FIG. 17), and for outputting the number of times of the movements/motions.

When observing nocturnal animals, fluorescence of a visible wavelength applied on the M1 and the M2 can be captured by using the capturing means 3 with the use of fluorescence as coating and by using the irradiating means 2 for irradiating with light as irradiating means of an ultraviolet ray such as a black light.

The capturing means 3 is a color digital image capturing device such as a CCD camera, and dynamic image data may be tentatively recorded by connecting a dynamic image recording means 11 such as a digital video equipment. The dynamic image is monitored by an image monitor 12. The capturing means 3, the dynamic image recording means 11, and the image monitor 12 are integrated in most of commercially available digital video cameras; therefore the cameras may be used.

The apparatus shown in FIG. 17 includes a display device 15 of a counting value (a display means for displaying the number of times of the movements/motions). The external setting input 16 shown in FIG. 17 is luminance or color condition data which is discriminating condition data, and the external setting input 17 shown in FIG. 17 is distance condition data which is motion determining condition data. An apparatus 30 shown in FIG. 17 comprises the apparatus for quantifying the specific movements/motions relating to the embodiment of this invention. This apparatus 30 may or may not include the monitor 12.

FIG. 18 is a view explaining the discriminating means 13 and the movement/motion counting means 14. These are explained in FIG. 18. Image data is inputted together with luminance data and color data of pixels of each image frame and coordinate data of the pixels from the capturing means 3 or the dynamic image recording means 11 to the discriminating means 13. Then, the color condition data decided in advance is inputted (an external setting input 16 in FIG. 18), and coordinates of the image data which fit conditions are extracted by comparing the condition data with the image data. This condition data may be a luminance condition or a combination of conditions of color and luminance.

As an easy extraction example, a condition that an R (red) data value is large is inputted as the condition data of the external setting input 16 when red coating is applied on the movement/motion tracking region. Then, the coordinate data of the pixels whose R (red) data value is larger than the condition data is selected and extracted from color data of the respective pixels of the image frame. This coordinate data is outputted to the movement/motion counting means 14.

The movement/motion counting means 14 includes a representative points deciding means 18 for deciding representative points (representative coordinates) in all of the coordinates which fit conditions of the image frame, a representative point distance calculating means 19 for calculating distances between the representative points decided by the representative points deciding means 18, and a movement/motion determining and counting means 20 for determining occurrence of the specific movements/motions from the representative point distance data calculated by the point distance calculating means 19 and the distance condition data decided in advance (an external setting input 17 in FIG. 18), and for counting the number of times of the movements/motions.

The representative points deciding means 18 extracts maximum values and minimum values of the respective coordinate axes among all of the coordinate data which fit the color or luminance condition data obtained from the discriminating means 13, calculates central values as the representative values from the aforementioned values, and outputs the coordinate data thereof. The discriminating means 13 can be any other means than that discriminating it by whether or not the value is larger/smaller than the aforementioned condition value such as color or luminance as described above. The representative points deciding means can be any other means than one deciding the central values of the coordinates as the representative values. Specifically, image processing methods such as an image differentiation method, a binarization method, an ellipse approximation, a centroid extraction, and the like may be adopted as necessary, so that discrimination and decision of the representative points are conducted based on these method.

The representative point distance calculating means 19 calculates the distances between the decided representative points in one image frame. The movement/motion determining and counting means 20 inputs the distances between the representative points calculated by the representative point distance calculating means 19, and the distance condition data decided in advance (the external setting input 17 in FIG. 18). For example, when the distance between the representative points are smaller than the condition data, the movement is considered to have occurred at the time the image frame was captured, and then a counter inside the movement/motion determining and counting means 20 counts up. Any methods for using the distance condition data can be applied to determinations of the movements. For example, when the value changes from a larger value than the condition value to a smaller one, they may be counted as one. As shown in FIG. 12, reciprocation from the larger value than the condition value to the smaller and back to the larger may be counted as one.

Numbers of the animal cages whose experimental images are captured, the time of each image frame, the distance between the representative points, and a storage address of the image data of the image frame may be stored as a set of data such as data of the cage number 1 and data of the cage number 2 as shown in FIG. 19 and FIG. 20 for convenience. The coordinates, the color data, and the like may be stored as a set in an image frame address, and cited as necessary. For example, if the data shown in FIG. 19 is considered as a data of a mouse to which a new medicine was administered in the cage number 1, and the data shown in FIG. 20 is considered as a data of a mouse to which a placebo was administered in the cage number 2, then the distance data of both of the mice for a certain same time can be instantaneously cited and easily compared, and the images at this time can be cited.

Observation time for processing quantification can be decided with no restraint by using the aforementioned data set, or the like. That is, if only the data set within the observation time is processed by the movement/motion determining and counting means 20, the data of the number of times of motions of the mouse to which the new medicine was administered and the mouse to which the placebo within the observation time was administered can be obtained, and it makes quantitative evaluation possible. Further, time intervals of the data collection can be also easily changed if the aforementioned data set is skipped over every three data set and used, or the like. Still more, actual situations in the image of the motions when the motions were counted can be checked by citing the image frame address. When many abnormal counts of the motions are found by this check, settings of the distance data for motion determining conditions are changed and then the counts of the motions may be reprocessed by the movement/motion determining and counting means 20.

In the above-mentioned manner, quantitative data of the movements/motions of the mouse to which the new medicine was administered and the mouse to which the placebo was administered can be conveniently obtained during the same period under various conditions. The new medicine can be evaluated under the condition of the highest reliability among the various conditions. Furthermore, in the case that an ordinary experiment must be redone due to necessity for a change of conditions, the embodiment of this invention allows easy change of the conditions, and results can be obtained without re-experiments.

The apparatus 30 shown in FIG. 17 is a part of the apparatus for quantifying the specific movements/motions relating to the embodiment of this invention. This quantification apparatus allows any marking methods such as the coating application described so far. That is, this quantification apparatus includes the discriminating means 13 of the marking areas for inputting the captured image data in which the organisms having plural movement/motion tracking regions marked and the discriminating condition data (the external setting input 16), and for discriminating the marking areas based on the image data and the discriminating condition data, and the movement/motion counting means 14 for determining the specific movements/motions from both the coordinate data of the discriminated marking area and the motion determining condition data (the external setting input 17), and for outputting the number of times of them.

FIG. 21 shows a display example of a monitor part of the quantification apparatus (a personal computer). This is a screen when processing the input dynamic images of the marked experimental animals. This screen includes a window Mo1 of a dynamic image monitor screen which is a replaying part of the image data of the cage number 1, and a window Mo2 of a dynamic image monitor screen which is a replaying part of an image data of the cage number 2. A displaying part Crgb displays color digital data (RGB values) of a position in the image pointed by a cursor Cs. A user can conveniently input conditions to a color condition data input part INP1 for discriminating the marking areas while watching the image of the window Mo1 of the color digital data.

This means that the color digital data (RGB values) at the position in the image pointed by the cursor Cs and which is certainly an extract part can be copied to the condition data input part INP1 (the external setting input 16 in FIG. 18). The same operation is to be performed even if the luminance data is used, while the color data is used in this example. Furthermore, the distance condition data (the external setting input 17 in FIG. 18) for determining the movements/motions, the time intervals of data collection, and times to start and to finish observation can be inputted to a condition data input part INP2 shown in FIG. 21. After these conditions are inputted, the discriminating means 13 and the movement/motion counting means 14 shown in FIG. 18 execute processing, and then a count value of the cage number 1 is displayed on a displaying part Ct1, and a count value of the cage number 2 on a displaying part Ct2.

The above quantification apparatus such as a personal computer or the like can process a captured image of the animals marked by any methods. In other words, an input interface card or the like for inputting the image data in which the organisms marked by any methods are captured can be equipped in the personal computer, and software for administering a man-machine interface described in FIG. 21 can be installed in the personal computer.

The discriminating means 13 in this apparatus, similarly to the above-mentioned means, is a means for extracting the coordinates of the image data which fit the conditions by comparing the luminance or color data of the captured image frame with the luminance condition data or the color condition data set up in the condition data input part INP1 or the like in advance, and for outputting the coordinates.

Furthermore, the movement/motion counting means 14, as mentioned above, includes the representative points deciding means 18 for deciding the representative points (the representative coordinates) in all of the coordinates which fit conditions in each of the image frames, the representative point distance calculating means 19 for calculating the distance between the representative points decided by the representative points deciding means 18, and the movement/motion determining and counting means 20 for determining occurrence of the specific movements/motions from the representative point distance data calculated by the representative point distance calculating means 19 and the distance condition data decided in advance by the condition data input part INP2 or the like (the external setting input 17 in FIG. 18), and for counting the number of times of the movements/motions.

This can provide the method for collecting data of the movements/motions without injury or stress in the animal experiments, and the simple and inexpensive method capable of objectively quantifying movements/motions based on the collected data. It also provides the method for collecting data of nocturnal animals such as mice, cats, bats, and the like in a dark environment where they behave naturally, and an apparatus for realizing it. Furthermore, it provides a convenient structure of the apparatus which allows change of the motion determining conditions and the like while watching the dynamic image freely and re-acquisition of quantitative data of the motions once the dynamic image data is captured.

Meanings of the numerals and symbols in the drawings are explained hereinafter.
1 ORGANISM TO WHICH COATING IS APPLIED SO AS TO INFINITELY COVER MOVEMENT/MOTION TRACKING REGIONS.
2 IRRADIATING MEANS FOR IRRADIATING ORGANISM WITH LIGHT, FOR EXAMPLE, VISIBLE LIGHT LAMP OR ULTRAVIOLET LAMP AND THE LIKE SUCH AS A BLACK LIGHT OR THE LIKE.
3 CAPTURING MEANS SUCH AS CCD CAMERA.
4 REPRESENTATIVE POINT DECIDED FROM COORDINATES OBTAINED BY DISCRIMINATING COATING-APPLIED AREAS (MARKING AREAS).
5 WIDTH BETWEEN MAXIMUM AND MINIMUM OF VERTICAL COORDINATES OBTAINED BY DISCRIMINATING COATING-APPLIED AREAS (MARKING AREAS).
6 LINE SHOWING CENTRAL POSITION COORDINATE OF WIDTH 5.
7 WIDTH BETWEEN MAXIMUM AND MINIMUM OF HORIZONTAL COORDINATES OBTAINED BY DISCRIMINATING COATING-APPLIED AREAS (MARKING AREAS).
8 LINE SHOWING CENTRAL POSITION COORDINATE OF WIDTH 7.
9 INJECTION SYRINGE FOR INJECTING IRRITANT.
10 INJECTION SITE OF THE INJECTION SYRINGE 9.
11 DYNAMIC IMAGE RECORDING MEANS SUCH AS A DIGITAL VIDEO EQUIPMENT.
12 IMAGE MONITOR.
13 DISCRIMINATING MEANS FOR DISCRIMINATING COATING-APPLIED AREAS (MARKING AREAS).
14 MOVEMENT/MOTION COUNTING MEANS FOR DECIDING MOVEMENTS/MOTIONS FROM IMAGE DATA AND CONDITION DATA, AND FOR OUTPUTTING NUMBER OF MOVEMENTS/MOTIONS.
15 DISPLAY DEVICE OF COUNTING VALUE (DISPLAY MEANS FOR DISPLAYING NUMBER OF MOVEMENTS/MOTIONS).
16 EXTERNAL SETTING INPUT OF LUMINANCE OR COLOR CONDITION DATA.
17 EXTERNAL SETTING INPUT OF DISTANCE CONDITION DATA.
18 REPRESENTATIVE POINTS DECIDING MEANS OF COATING-APPLIED AREAS (MARKING AREAS).
19 REPRESENTATIVE POINT DISTANCE COUNTING MEANS.
20 MOVEMENT/MOTION DETERMINING AND MOTION TIMES COUNTING MEANS.
30 APPARATUS FOR QUANTIFYING SPECIFIC MOVEMENTS/MOTIONS RELATING TO AN EMBODIMENT OF THIS INVENTION.
   Crgb DISPLAYING PART OF COLOR DIGITAL DATA (RGB VALUES) OF PORTION POINTED BY CURSOR Cs.
Cs CURSOR.
Ct1 DISPLAYING PART OF COUNTING VALUE OF CAGE NUMBER 1.
Ct2 DISPLAYING PART OF COUNTING VALUE OF CAGE NUMBER 2
   Data DATA PLOT OF DISTANCE (INTERVAL) BETWEEN REPRESENTATIVE POINTS AT CERTAIN TIME
   INP1 INPUT PART OF COLOR CONDITION DATA FOR DISCRIMINATING MARKING AREAS
   INP2 INPUT PART OF DISTANCE CONDITION DATA (Th), TIME INTERVAL OF DATA COLLECTION AND OBSERVATION TIME
   M1 PORTION WHERE COATING IS APPLIED SO AS TO INFINITELY COVER HEAD
   M2 PORTION WHERE COATING IS APPLIED SO AS TO INFINITELY COVER REAR LEG
   M3 PORTION WHERE COATING IS APPLIED SO AS TO INFINITELY COVER MOUTH
   M4 PORTION WHERE COATING IS APPLIED SO AS TO INFINITELY COVER INJECTION SITE OF IRRITANTS FOR INDUCING PAIN
   M5 PORTION WHERE COATING IS APPLIED SO AS TO INFINITELY COVER NOSE TO WHICH IRRITANT IS APPLIED
   M6 PORTION WHERE COATING IS APPLIED SO AS TO INFINITELY COVER FRONT LEGS
   Mo1 REPLAYING PART OF IMAGE DATA OF CAGE NUMBER 1
   Mo2 REPLAY ING PART OF IMAGE DATA OF CAGE NUMBER 2
   Th LEVEL SHOWING DISTANCE CONDITION DATA DECIDED IN ADVANCE (THRESHOLD LEVEL)
   x SYMBOL SHOWING REPRESENTATIVE POINT LOCATIONS OF MARKING AREAS ON HEAD OF MOUSE
   + SYMBOL SHOWING REPRESENTATIVE POINT LOCATIONS OF MARKING AREAS ON TOP OF REAR LEGS OF MOUSE

### Industrial Availability

According to this invention, in biological experiments such as pharmaceutical tests or the like, foreign substances are not attached to organisms, resulting in no stress to the organisms. Furthermore, nocturnal animals can be captured in a dark place; therefore, the organisms behave naturally and the experiments can ensure high objectivity and reliability. The organisms suffer little injury because preparation of marking is easy and because instruments such as a vibration sensor, a magnetic sensor, and the like are not attached to the organisms. A chamber with a detection device is not required, either. Objective quantification of animal behaviors is automated, and then less human resources for determining the behaviors are required, and there is no need for demanding labor such as constant monitoring by observers or the like.

Processing time is short and practical because quantitative data can be obtained through extremely simple calculation which is a calculation of distance between representative points by extracting only marking areas and by calculating their representative points, rather than the behaviors are decided by thoroughly processing all image data one by one in dynamic image processing. The dynamic image can be replayed; furthermore, conditions of extraction of the marking areas, conditions of motion determining decisions, and those of distance determining decisions can be conveniently changed and inputted while watching the dynamic image. Such change of the conditions can be repeatedly reprocessed by using the same dynamic image data, which dramatically decreases frequency of re-experiments.

Still more, a unique effect that standard of quantifying for determining movements/motions can be specified based on optimum determining conditions can be also expected because the optimum determining conditions with high objectivity and reliability in relation to the specific movement/motion can be exploratorily sought while changing the conditions.

Explanation is given by employing the pharmaceutical tests on the animals; however, this invention can be also applied to general biological experiments and biological tests on insects, fish, and the like. Application to clinical trials or the like on humans are excepted.

## Claims

1. A method for quantifying specific movement/motion of an organism by applying coating which includes a dye or a fluorescent material to plural movement/motion tracking regions on surface of the organism decided based on the movement/motion of the organism, the method for automating observation of the movement/motion of the organism comprising:
a first step for deciding an affected part of the organism and a portion with which the organism acts on the affected part as the movement/motion tracking regions;
a second step for marking the movement/motion tracking regions by applying the coating which includes a dye or a fluorescent material;
a third step for capturing an image of the organism under light irradiation;
a fourth step for discriminating marking areas in a captured image and for deciding representative point locations of the marking areas; and
a fifth step for determining the specific movement/motion based on representative point location data and for outputting the number of times of the movement/motion.

2. The method for automating observation of the movement/motion of the organism according to claim 1, wherein
the organism is a model animal which naturally develops dermatitis;
the specific movement/motion is scratching behavior;
the affected part is from a head to a back; and
the portion of the organism with which the organism acts on the affected part is a front leg or a rear leg.

3. The method for automating observation of the movement/motion of the organism according to claim 1 comprising:
applying an irritant to the affected part or administering an irritant to the organism to induce the specific movement/motion by stimulating the affected part.

4. The method for automating observation of the movement/motion of the organism according to claim 3, wherein
the organism is a model animal which develops dermatological illness;
the specific movement/motion is scratching behavior;
the irritant is an irritant for inducing itch; and
the portion of the organism with which the organism acts on the affected part is a front leg or a rear leg.

5. The method for automating observation of the movement/motion of the organism according to claim 3, wherein
the organism is a model animal which develops otolaryngologic illness or ophthalmic illness;
the specific movement/motion is sneeze or sniffle behavior;
the irritant is an irritant for inducing the sneeze or sniffle behavior; and
the portion of the organism with which the organism acts on the affected part is a front leg or a rear leg.

6. The method for automating observation of the movement/motion of the organism according to claim 3, wherein
the organism is a model animal of aching illness relating internal medicine and/or surgery;
the specific movement/motion is biting behavior of surface of its own body;
the irritant is an irritant for inducing pain; and
the portion of the organism with which the organism acts on the affected part is a mouth.

7. The method for automating observation of the movement/motion of the organism according to claim 3, wherein
the organism is a model animal of aching illness relating internal medicine and/or surgery;
the specific movement/motion is scratching behavior of surface of its own body;
the irritant is an irritant for inducing pain; and
the portion of the organism with which the organism acts on the affected part is a front leg or a rear leg.

8. The method for automating observation of the movement/motion of the organism according to any one of claims 1 to 7, wherein
the organism is an nocturnal animal; and
said third step is conducted under irradiation with ultraviolet light.

9. The method for automating observation of the movement/motion of the organism according to any one of claims 1 to 8, wherein
said fifth step is a step for calculating distance between the representative points based on the plural representative point location data, and for determining whether the specific movement/motion occurred or not by the distance.

10. An apparatus for quantifying specific movement/motion of an organism by applying coating which includes a dye or a fluorescent material to plural movement/motion tracking regions on surface of the organism decided based on the movement/motion, the apparatus for automating observation of the movement/motion of the organism comprising:
an irradiating means for irradiating, with light, the organism to which the coating including a dye or a fluorescent material is applied on both an affected part of the organism and a portion of the organism with which the organism acts on the affected part, decided as the movement/motion tracking regions;
a capturing means for capturing an image of the organism under said irradiating means;
a discriminating means for discriminating coating-applied areas from both captured image data and discriminating condition data; and
a movement/motion counting means for determining the specific movement/motion from both coordinate data of the discriminated coating-applied areas and motion determining condition data, and for outputting the number of times of the movement/motion.

11. The apparatus for automating observation of the movement/motion of the organism according to claim 10, wherein
the organism is a nocturnal animal; and
said irradiating means is an irradiating means of ultraviolet light.

12. The apparatus for automating observation of the movement/motion of the organism according to claim 10 or claim 11, wherein
said discriminating means is a means for inputting captured image data, and luminance condition data or color discriminating condition data decided in advance, for comparing these data, and for extracting coordinates of the image data which fit conditions as coordinate data of the coating-applied areas; and
said movement/motion counting means is a means for inputting the coordinate data of the discriminated coating-applied areas and the motion determining distance condition data decided in advance, for deciding representative point coordinates of the coating-applied areas from the coordinate data of the coating-applied areas, for calculating distance between the representative points from representative point coordinate data, for determining occurrence of the specific movement/motion from the distance between the representative points and distance condition data, and for counting the number of times of the movement/motion.

13. An apparatus for quantifying specific movement/motion of an organism by marking plural movement/motion tracking regions on surface of an organism decided based on the movement/motion, the apparatus for quantifying the specific movement/motion of the organism comprising:
a discriminating means of marking areas for discriminating the marking areas based on discriminating condition data and captured image data in which the organism marked on its surface decided as movement/motion tracking regions; and
a movement/motion counting means for determining the specific movement/motion based on coordinate data of the discriminated marking areas and motion determining condition data, and for outputting the number of times of the movement/motion.

14. The apparatus for quantifying the specific movement/motion of the organism according to claim 13, wherein
said discriminating means is a means for inputting the image data, and luminance condition data or color discriminating condition data decided in advance, for comparing these data, and for extracting coordinates of the image data which fit conditions as coordinate data of the marking areas; and
said movement/motion counting means is a means for inputting the coordinate data of the discriminated marking areas and motion determining distance condition data decided in advance, for deciding representative point coordinates of the marking areas from the coordinate data of the marking areas, for calculating distance between representative points from the representative point coordinate data, for determining occurrence of the specific movement/motion from the distance between the representative points and the distance condition data, and for counting the number of times of the movement/motion.

## Patentansprüche

1. Verfahren zum Quantifizieren spezifischer Bewegungen/Körperbewegungen eines Organismus durch Aufbringen einer Beschichtung, die eine Farbe oder ein fluoreszentes Material enthält, auf mehrfache Nachverfolgungsbereiche für Bewegung/Körperbewegung des bestimmten Organismus, basierend auf der Bewegung/Körperbewegung des Organismus, wobei das Verfahren zum automatischen Beobachten der Bewegung/Körperbewegung des Organismus folgendes aufweist:
Einen ersten Schritt zum Bestimmen eines betroffenen Teils des Organismus und eines Bereichs, mit dem der Organismus auf den betroffenen Teil agiert als Nachverfolgungsbereiche für die Bewegung/Körperbewegung;
einen zweiten Schritt zum Markieren der Nachverfolgungsbereiche der Bewegung/Körperbewegung durch Aufbringen der Beschichtung, die eine Farbe oder ein fluoreszierendes Material enthält;
einen dritten Schritt zum Aufnehmen eines Bildes des Organismus unter Lichtbestrahlung;
einen vierten Schritt zum Unterscheiden von Markierungsgebieten in einem aufgenommenen Bild und zum Bestimmen von Orten repräsentativer Punkte der Markierungsgebiete; und
einen fünften Schritt zum Bestimmen der spezifischen Bewegung/Körperbewegung basierend auf Daten von Orten repräsentativer Punkte und zum Ausgeben der Anzahl von Bewegungen/Körperbewegungen.

2. Verfahren zum Automatisieren von Beobachtungen der Bewegung/Körperbewegung des Organismus nach Patentanspruch 1, wobei
der Organismus ein Versuchstier ist, das auf natürliche Weise Dermatitis entwickelt;
die spezifische Bewegung/Körperbewegung ein Kratz-Verhalten ist;
der betroffene Teil sich vom Kopf zum Rücken erstreckt; und
der Teil des Organismus, mit dem der Organismus auf den betroffenen Teil agiert, ein Vorder-oder Hinterbein ist.

3. Verfahren zum Automatisieren von Beobachtungen der Bewegung/Körperbewegung des Organismus nach Patentanspruch 1, umfassend:
Anwenden eines Reizmittels auf das betroffene Teil oder Lenken eines Reizmittels auf den Organismus zum Induzieren der spezifischen Bewegung/Körperbewegung durch Stimulieren des beeinflussten Teils.

4. Verfahren zum Automatisieren von Beobachtungen der Bewegung/Körperbewegung des Organismus nach Patentanspruch 3, wobei
der Organismus ein Versuchstier ist, das dermatologische Krankheit entwickelt;
die spezifische Bewegung/Körperbewegung ein Kratz-Verhalten ist;
das Reizmittel ein Juckreizmittel ist; und
der Teil des Organismus, mit dem der Organismus auf den beeinflussten Teil agiert, ein Vorder-oder ein Hinterbein ist.

5. Verfahren zum Automatisieren von Beobachtungen der Bewegung/Körperbewegung des Organismus nach Patentanspruch 3, wobei
der Organismus ein Versuchstier ist, das otolaryngologische Krankheit oder ophthalmische Krankheit entwickelt;
die spezifische Bewegung/Körperbewegung ein Nies- oder Schniefverhalten ist;
das Reizmittel ein solches ist, welches das Nies-oder Schniefverhalten auslöst; und
der Teil des Organismus, mit dem der Organismus auf das beeinflusste Teil agiert, ein Vorder-oder ein Hinterbein ist.

6. Verfahren zum Automatisieren von Beobachtungen der Bewegung/Körperbewegung des Organismus nach Patentanspruch 3, wobei
der Organismus ein Versuchstier mit schmerzhafter Krankheit ist, die auf innere Medizin und/oder Chirurgie zurückgeht;
die spezifische Bewegung/Körperbewegung ein Beiß-Verhalten auf die Außenfläche seines eigenen Körpers ist;
das Reizmittel ein solches ist, das Schmerz auslöst; und
der Teil des Organismus, mit dem der Organismus auf das betroffene Teil agiert, ein Mund ist.

7. Verfahren zum Automatisieren von Beobachtungen der Bewegung/Körperbewegung des Organismus nach Patentanspruch 3, wobei
der Organismus ein Versuchstier mit schmerzhafter Krankheit ist, die auf innere Medizin und/oder Chirurgie zurückgeht;
die spezifische Bewegung/Körperbewegung ein Kratzverhalten der Außenfläche seines eigenen Körpers ist;
das Reizmittel ein solches ist, das Schmerz auslöst; und
der Teil des Organismus, mit dem der Organismus auf das beeinflusste Teil agiert, ein Vorder-oder Hinterbein ist.

8. Verfahren zum Automatisieren von Beobachtungen der Bewegung/Körperbewegung des Organismus nach irgendeinem der Patentansprüche 1 bis 7, wobei
der Organismus ein Nachttier ist; und
der besagte dritte Schritt unter Bestrahlung mit ultraviolettem Licht ausgeführt wird.

9. Verfahren zum Automatisieren von Beobachtungen der Bewegung/Körperbewegung des Organismus nach irgendeinem der Patentansprüche 1 bis 8, wobei
der besagte fünfte Schritt ein Schritt zum Berechnen des Abstands zwischen den repräsentativen Punkten ist, basierend auf den Ortsdaten der vielfachen repräsentativen Punkte, und zum Bestimmen, ob die spezifische Bewegung/Körperbewegung in dem Abstand auftrat oder nicht.

10. Gerät zum Quantifizieren spezifischer Bewegung/Körperbewegung eines Organismus durch Aufbringen einer Beschichtung, die eine Farbe oder ein fluoreszierendes Material enthält, auf vielfache Nachverfolgungsbereiche der Bewegung/Körperbewegung auf die Außenfläche des bestimmten Organismus, basierend auf der
Bewegung/Körperbewegung, wobei das Gerät zum automatischen Beobachten der Bewegung/Körperbewegung des Organismus umfasst:
Bestrahlungsmittel zum Bestrahlen des Organismus mit Licht, auf den die Beschichtung mit Farbe oder einem fluoreszierenden Material sowohl auf ein beeinflusstes Teil des Organismus als auch auf einen Teil des Organismus, mit dem der Organismus auf das betroffene Teil agiert, aufgebracht ist, welche als die Bereiche der Bewegung/Körperbewegung bestimmt sind;
Aufnahmemittel zum Aufnehmen eines Bildes des Organismus unter dem Bestrahlungsmittel;
Unterscheidungsmittel zum Unterscheiden von beschichteten Gebieten aus Daten aufgenommener Bilder und aus Daten unterschiedener Zustände; und
Zählmittel der Bewegung/Körperbewegung zum Bestimmen der spezifischen Bewegung/Körperbewegung sowohl aus Koordinatendaten der unterschiedenen beschichteten Bereiche wie auch der die Bewegung festlegenden Zustandsdaten, und zum Ausgeben der Anzahl von Bewegungen/Körperbewegungen.

11. Gerät zum Automatisieren von Beobachtungen der Bewegung/Körperbewegung des Organismus nach Patentanspruch 10, wobei
der Organismus ein Nachttier ist; und
das besagte Bestrahlungsmittel eine Bestrahlungseinrichtung mit ultraviolettem Licht ist.

12. Gerät zum Automatisieren von Beobachtungen der Bewegung/Körperbewegung des Organismus nach Patentanspruch 10 oder 11, wobei
das Unterscheidungsmittel eine Einrichtung zum Eingeben der Daten aufgenommener Bilder, sowie von voraus festgelegten Luminanz-Zustandsdaten oder Farbdiskriminier-Zustandsdaten ist, zum Vergleichen dieser Daten und zum Extrahieren von Koordinaten der Bilddaten, welche Bedingungen als Koordinatendaten der beschichteten Bereiche erfüllen; und
das genannte Bewegungs-/Körperbewegungs-Zählmittel eine Einrichtung zum Eingeben der Koordinatendaten der unterschiedenen beschichteten Gebiete und der voraus festgelegten, die Bewegung bestimmenden Abstandszustandsdaten ist, zum Bestimmen der Koordinaten repräsentativer Punkte der beschichteten Bereiche aus den Koordinatendaten der beschichteten Bereiche, zum Berechnen des Abstands zwischen den repräsentativen Punkten aus den Koordinatendaten der repräsentativen Punkte, zum Bestimmen des Auftretens der spezifischen Bewegung/Körperbewegung aus dem Abstand zwischen den repräsentativen Punkten und den Abstandszustandsdaten, sowie zum Zählen der Anzahl von Bewegungen/Körperbewegungen.

13. Gerät zum Quantifieren der spezifischen Bewegung/Körperbewegung eines Organismus durch Markieren vielfacher Nachverfolgungsbereiche von Bewegung/Körperbewegung auf der Außenfläche eines bestimmten Organismus, basierend auf der Bewegung/Körperbewegung, wobei das Gerät zum Quantifizieren der spezifischen Eigen-/Antriebsbewegung des Organismus enthält:
Unterscheidungsmittel von Markierungsgebieten zum Unterscheiden der Markierungsgebiete, basierend auf Unterscheidungszustandsdaten und Daten aufgenommener Bilder, worin der Organismus auf seiner Oberfläche markiert ist, die als Regionen von Bewegung/Körperbewegung bestimmt ist; und
Zählmittel für Bewegung/Körperbewegung zum Bestimmen der spezifischen Bewegung/Körperbewegung, basierend auf Koordinatendaten der unterschiedenen Markierungsgebiete und bewegungsbestimmender Zustandsdaten, sowie zum Ausgeben der Anzahl von Bewegungen/Körperbewegungen.

14. Gerät zum Quantifieren der spezifischen Bewegung/Körperbewegung des Organismus nach Patentanspruch 13, wobei
die genannten Unterscheidungsmittel eine Einrichtung zum Eingeben der Bilddaten, sowie der voraus festgelegten Luminanzzustandsdaten oder Farbdiskriminier-Zustandsdaten ist, zum Vergleichen dieser Daten und zum Extrahieren von Koordinaten der Bilddaten, die zu Bedingungen als Koordinatendaten der Markierungsgebiete passen; und das genannte Bewegungs-/Körperbewegungs -Zählmittel eine Einrichtung zum Eingeben der Koordinatendaten der voraus festgelegten, diskriminierten Markierungsgebiete und der bewegungsbestimmenden Abstandszustandsdaten ist, zum Bestimmen der Koordinaten repräsentativer Punkte der Markierungsgebiete aus den Koordinatendaten der Markierungsgebiete, zum Berechnen des Abstands zwischen repräsentativen Punkten aus den Koordinatendaten der repräsentativen Punkte, zum Bestimmen des Auftretens der spezifischen Bewegung/Körperbewegung aus dem Abstand zwischen den repräsentativen Punkten und den Abstandszustandsdaten, und zum Zählen der Anzahl von Bewegungen/Körperbewegungen.

## Revendications

1. Un procédé pour quantifier un geste/mouvement spécifique d'un organisme en appliquant une couche comportant un colorant ou un matériau fluorescent sur plusieurs zones de suivi de geste/mouvement sur la surface de l'organisme sélectionné en se basant sur le geste/mouvement de l'organisme, le procédé servant à automatiser l'observation du geste/mouvement de l'organisme comprenant :
une première étape pour définir comme zones de suivi de geste/mouvement une partie affectée de l'organisme et une portion avec laquelle l'organisme agit sur la partie affectée;
une deuxième étape pour marquer des zones de suivi de geste/mouvement en appliquant la couche comportant un colorant ou un matériau fluorescent;
une troisième étape pour capturer une image de l'organisme sous irradiation lumineuse;
une quatrième étape pour distinguer les zones de marquage sur une image capturée et pour décider des localisations des points représentatifs des zones de marquage; et
une cinquième étape pour déterminer le geste/mouvement spécifique basé sur les données de la localisation du point représentatif et pour collecter les informations sur le nombre de gestes/déplacements.

2. Le procédé pour automatiser l'observation du geste/mouvement de l'organisme selon la revendication 1 où
l'organisme est un animal modèle qui développe naturellement une dermite;
le geste/mouvement spécifique est un comportement de grattage;
la partie affectée va de la tête au dos; et
la portion de l'organisme avec laquelle l'organisme agit sur la partie affectée est une patte avant ou une patte arrière.

3. Le procédé pour automatiser l'observation du geste/mouvement de l'organisme selon la revendication 1 comprenant :
l'application d'un irritant sur la partie affectée ou l'administration d'un irritant à l'organisme pour provoquer le geste/mouvement spécifique en stimulant la partie affectée.

4. Le procédé pour automatiser l'observation du geste/mouvement de l'organisme selon la revendication 3 où
l'organisme est un animal modèle qui développe une pathologie dermatologique;
le geste/mouvement spécifique est un comportement de grattage;
l'irritant est un irritant provoquant une démangeaison;
la portion de l'organisme avec laquelle l'organisme agit sur la partie affectée est une patte avant ou une patte arrière.

5. Le procédé pour automatiser l'observation du geste/mouvement de l'organisme selon la revendication 3 où
l'organisme est un animal modèle qui développe une pathologie oto-laryngologique ou ophtalmique;
le geste/mouvement spécifique est un comportement d'éternuement ou de reniflement;
l'irritant est un irritant provoquant un éternuement ou un reniflement; et
la portion de l'organisme avec laquelle l'organisme agit sur la partie affectée est une patte avant ou une patte arrière.

6. Le procédé pour automatiser l'observation du geste/mouvement de l'organisme selon la revendication 3 où
l'organisme est un animal modèle qui développe une pathologie douloureuse relevant de la médecine interne et/ou de la chirurgie;
le geste/mouvement spécifique est un comportement de morsure à la surface de son propre corps;
l'irritant est un irritant provoquant la douleur; et
la portion de l'organisme avec laquelle l'organisme agit sur la partie affectée est la gueule.

7. Le procédé pour automatiser l'observation du geste/mouvement de l'organisme selon la revendication 3 où
l'organisme est un animal modèle qui développe une pathologie douloureuse relevant de la médecine interne et/ou de la chirurgie;
le geste/mouvement spécifique est un comportement de grattage à la surface de son propre corps;
l'irritant est un irritant provoquant la douleur; et
la portion de l'organisme avec laquelle l'organisme agit sur la partie affectée est une patte avant ou une patte arrière.

8. Le procédé pour automatiser l'observation du geste/mouvement de l'organisme selon l'une quelconque des revendications 1 à 7 ou
l'animal est un animal nocturne;
la troisième étape précitée est réalisée sous irradiation de lumière ultraviolette.

9. Le procédé pour automatiser l'observation du geste/mouvement de l'organisme selon l'une quelconque des revendications 1 à 8 ou
la cinquième étape précitée est une étape permettant de calculer la distance entre les points représentatifs en se basant sur les données de la localisation des multiples points représentatifs, et de déterminer selon la distance si le geste/mouvement spécifique a été effectué ou non.

10. Un appareil pour quantifier les gestes/mouvements spécifiques de l'organisme en appliquant une couche comportant un colorant ou un matériau fluorescent sur plusieurs zones de suivi de geste/mouvement sur la surface de l'organisme sélectionné en se basant sur le geste/mouvement de l'organisme, l'appareil servant à automatiser l'observation du geste/mouvement de l'organisme comprenant :
un moyen d'irradier à l'aide de lumière l'organisme sur lequel a été appliqué la couche comportant un colorant ou un matériau fluorescent à la fois sur une partie affectée de l'organisme et une portion de l'organisme avec laquelle l'organisme agit sur la partie affectée, définies comme zones de suivi de geste/mouvement;
un moyen de capture pour capturer une image de l'organisme sous l'irradiation précitée;
un moyen de distinction pour distinguer les zones sur lesquelles la couche a été appliquée à la fois sur les données des images capturée et sur les données des conditions distinctives; et
un moyen de comptage des gestes/mouvements pour déterminer les gestes/mouvements spécifiques des données coordonnées à la fois des zones distinctes sur lesquelles la couche a été appliquée et les données de condition définissant le mouvement, et pour relever le nombre de répétitions du geste/mouvement.

11. L'appareil pour l'observation automatisée d'un geste/mouvement de l'organisme selon la revendication 10 où
l'organisme est un animal nocturne; et
le moyen d'irradiation précité est un moyen d'irradiation à lumière ultraviolette.

12. L'appareil pour l'observation automatisée d'un geste/mouvement de l'organisme selon la revendication 10 ou 11 où
le moyen distinctif précité est un moyen de saisir les données de l'image capturée, et les données sur les conditions de luminance ou les données sur les conditions de distinction des couleurs décidées par avance, de comparer ces données, et d'extraire les coordonnées des données des images qui correspondent aux conditions des données des coordonnées des zones sur lesquelles a été appliqué la couche; et
le moyen de comptage des gestes/mouvements précité est un moyen de saisir les données des coordonnées des zones distinctes sur lesquelles a été appliqué la couche et les données des conditions de distance déterminant le mouvement décidées par avance, de décider des coordonnées des points représentatifs des zones sur lesquelles a été appliqué la couche des données de coordonnées des zones sue lesquelles a été appliqué la couche, de calculer la distance entre les points représentatifs des données de coordonnées des points représentatifs pour déterminer si le geste/mouvement spécifique a été effectué grâce à la distance entre les points représentatifs et les données de conditions de distance, et de compter le nombre de répétitions du geste/mouvement.

13. L'appareil pour quantifier le geste/mouvement spécifique de l'organisme en marquant plusieurs zones de suivi de geste/mouvement à la surface de l'organisme sélectionné en se basant sur le geste/mouvement de l'organisme, l'appareil pour quantifier les gestes/mouvements spécifiques comprenant :
un moyen distinctif des zones de marquage pour distinguer les zones de marquage en se basant sur les données de condition de distinction et les données de l'image capturées dans laquelle se trouvent les zones de suivi de geste/mouvement sélectionnées sur l'organisme marqué; et
un moyen de comptage des gestes/mouvements pour déterminer le geste/mouvement spécifique se basant sur des données coordonnées des zones de marquage distinctes et des données de conditions définissant le mouvement, et pour obtenir le nombre de répétitions du geste/mouvement.

14. L'appareil pour quantifier les gestes/mouvements spécifiques de l'organisme selon la revendication 13 ou
le moyen distinctif précité est un moyen pour saisir les données de l'image, et les données de la condition de luminance ou les données de la condition de distinction des couleurs décidées à l'avance, pour comparer ces données, et pour extraire des coordonnées des données des images qui correspondent aux conditions en tant que données coordonnées des zones de marquage; et
le moyen de comptage des gestes/mouvements précité est un moyen pour saisir les données coordonnées des zones de marquage distinguées et les données de condition de distance définissant le mouvement décidées par avance, pour décider de coordonnées de points représentatifs des zones de marquage des données de coordonnées des zones de marquage, pour calculer la distance entre des points représentatifs des données de coordonnées de points représentatifs, pour déterminer selon la distance entre les points représentatifs et les données de condition de distance si le geste/mouvement spécifique a été effectué, et pour compter le nombre de répétition du geste/mouvement.
